# DEMANDE DE BREVET EUROPEEN

(11) **EP 1 097 714 A1**
(43) Date de publication de la demande: **09.05.2001**
(21) Numéro de dépôt: 00122667.9
(22) Date de dépôt: 21.02.1994
(51) Int. Cl.: A61K 35/12, A61K 35/74, A61K 35/76, A61K 48/00, A61K 38/45, A61P 35/00

(54) **Composition pour le traitement de tumeurs et l'immunisation d'organismes à l' encontre de tumeurs**

(30) Priorité: 22.02.1993 FR 9302012
(62) Demande divisionnaire de: 94907603.8
(71) Demandeur: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR); UNIVERSITE PIERRE ET MARIE CURIE PARIS VI, 75252 Paris Cédex 05 (FR); ASSISTANCE PUBLIQUE, HOPITAUX DE PARIS, 75004 Paris (FR)
(72) Inventeur: Roth, Claude, 75116 Paris (FR); Kourilsky, Philippe, 75001 Paris (FR); Mir, Luis, 91370 Verrières le Buisson (FR); Klatzmann, David, 75013 Paris (FR); Salzmann, Jean-Loup, 75005 Paris (FR)
(74) Mandataire: Becker, Philippe

(57) **Abrégé**

Composition destinée à traiter les tumeurs d'organismes humains ou animaux et à immuniser ces organismes à l'encontre des tumeurs, ladite composition comprenant en association synergique :
- des cellules, des virus, ou des bactéries exprimant de manière transitoire dans l'organisme au moins un gène leur permettant de produire in vivo un ou plusieurs immunomodulateurs , et
- des virus , ou des cellules produisant des virus, lesdits virus infectant préférentiellement les cellules en division de l'organisme traité et portant dans leur génome au moins un gène dont l'expression dans les cellules en division va entraîner leur mort .

## Description

La présente invention a pour objet une composition pour le traitement de tumeurs et l'immunisation d'organismes à l'encontre de tumeurs.

Il a été établi très récemment, par diverses équipes scientifiques, que l'injection localisée dans des organismes , atteints par une tumeur, de cellules tumorales syngéniques produisant une interleukine permettait le rejet de cette tumeur par l'organisme.

Ceci a été mis en évidence pour l'interleukine-2 par Bubenik et al. ( Immunology Letters, 19, 279-282, 1988; Immunology Letters, 23, 287-292, 1989) et confirmé notamment par Fearon et al. (Cell., 60, 397-403, 1990) et par Ley et al., (European Journal of Immunology 1991, 21 : 851-854; Res. Immunol., 1990, 141: 855-863).

Les auteurs de ces articles mentionnent que le rejet s'accompagne d'une mémorisation de la réponse. L'animal est ainsi vacciné contre le développement ultérieur d'une tumeur d'un même type, même si celle-ci a été greffée sur un site différent .

Des cellules cancéreuses syngéniques produisant l'Interleukine-4 ont aussi été testées avec des résultats semblables , comme le rapportent Golumbek (Science, 254, 713 - 716, 1991) et Tepper et al. (Cell, 57, 503-512, 1989) ainsi que des cellules produisant le facteur de nécrose des tumeurs (TNF) comme le décrit Blankenstein et al. ( J. Exp. Med., 173, 1047-1052, 1991).

Il a aussi été évoqué (Pardoll, Current Opinion in Oncology, Vol.4, N°6, 1124-1129, 1992) la possibilité de co-introduire dans des cellules tumorales issues de l'organisme à traiter, d'une part des gènes codant pour des cytokines et, d'autre part, des gènes suicides tels que le gène de la thymidine-kinase du virus de l'herpès (HSVTK).

L'auteur mentionne que cette stratégie est particulièrement compliquée et nécessiterait la transduction de 100% des cellules.

Cette stratégie a néanmoins été testée dans la demande PCT/US 91/06 612 au nom de THE JOHN HOPKINS UNIVERSITY et THE UNIVERSITY OF TEXAS SYSTEM qui a pour objet des compositions destinées à potentialiser la réponse immunitaire à l'encontre d'une tumeur, comprenant des cellules issues de cette tumeur:
- qui expriment un polypeptide immunopotentialisateur , et
- possèdent un gène susceptible de tuer ces cellules, ou gène suicide.

Le polypeptide immunopotentialisateur peut être une cytokine telle qu'une interleukine 1 ou 2. Le gène suicide peut être par exemple le gène de la thymidine-kinase.

Les compositions cellulaires objets de cette demande sont issues de l'organisme à traiter et sont donc syngéniques pour cet organisme. Elles ne comprennent pas de virus.

Les systèmes décrits dans ces publications présentent des inconvénients en vue de leur application à l'homme .

En effet, dans toutes ces publications, les cellules produisant les interleukines sont des cellules de l'individu ou d'un individu syngénique , qui ont été modifiées afin qu'elles expriment l'interleukine.

Dans le cas d'une thérapie humaine , un inconvénient de cette méthodologie réside dans le fait que les cellules exprimant l'interleukine et injectées à l'organisme risquent de continuer à se développer même après le rejet de la tumeur .

Afin de pallier cet inconvénient , une méthode de traitement thérapeutique a été testée , qui consiste à injecter à l'organisme des cellules allogéniques ou xénogéniques exprimant des gènes leur permettant de produire in vivo une ou plusieurs substances biologiquement actives, telles que de l'Interleukine-2 (voir la demande de brevet FR 91 14 119 du 15 Novembre 1991 intitulée " Composition cellulaire pour le traitement des organismes humains ou animaux") .

Cette méthode permet un traitement transitoire de l'organisme par ces substances , car les cellules , du fait de leur nature immunologique, sont rejetées par l'organisme .

Ce traitement a été testé par injection à proximité de cellules tumorales ( tumeur LPB ) de cellules allogéniques produisant de l'Interleukine-2, 9 jours après inoculation par les cellules tumorales . On observe un effet bénéfique se traduisant par un ralentissement de la croissance tumorale sur quelques jours .

Cet effet est transitoire et ne permet pas toujours, dans les conditions utilisées , d'induire une mémorisation immunitaire spécifique de la cellule tumorale inoculée. En effet, chez les animaux ainsi traités , l'inoculation ultérieure de la même cellule tumorale ( tumeur de Lewis ) peut se traduire par une croissance tumorale .

En outre, l'effet observé , c'est-à-dire le ralentissement de la croissance tumorale , n'est pas toujours suffisant pour provoquer l'élimination dans l'ensemble de la masse tumorale .

On remarquera que , dans toutes les expériences décrites dans l'état de la technique , on mesure généralement l'absence de croissance tumorale sur un animal sain et très rarement sur des tumeurs préétablies . Ainsi , GOLUMBEK et al,( ( 1991) Science, 254, 713-716) et PORGADOR et al.( (1992) Cancer Res. 52; 3679-3686) , ont dans les deux cas injecté une composition dans le but de traiter une tumeur préétablie , mais au moment du traitement la tumeur préétablie n'était ni visible ni décelable macroscopiquement.

Dans une approche différente TROJAN et al., ((1992) Proc. Natl. Acad. Sci., USA, 89, 4874-4878 , (1993) Science, 259, 94-97) ont modifié l'immunogénicité d'une tumeur chez le rat ( gliome) en transfectant les cellules tumorales avec un vecteur codant pour un DNA complémentaire antisens de l'IGF1 (Insulin-like Growth Factor 1). Les auteurs mentionnent que l'injection de ces cellules modifiées se traduit par l'absence de tumorigénicité et par un effet à distance sur une tumeur préétablie Néanmoins, cette approche est limitée à des cellules tumorales sécrétant de l'IGF comme facteur autocrine de croissance et elle nécessite une manipulation de chaque cellule tumorale pour générer une réponse immunitaire spécifique .

Une autre proposition basée sur l'infection de cellules tumorales par un rétrovirus portant le gène codant pour la thymidine kinase de l'herpès a été testée sur deux modèles. Dans le premier modèle Culver et al. ((1992) Science, 256, 1550-1552) développent une approche thérapeutique dans laquelle on injecte, dans un gliome ( tumeur du cerveau) de rat, des cellules xénogéniques produisant un vecteur rétroviral, dans lequel le gène codant pour la thymidine kinase de l'herpès a été inséré. Après production locale de particules virales TK⁺ , qui vont infecter les cellules à croissance rapide ( cellules tumorales ) , le traitement systémique par le Ganciclovir ( Merck Index, référence 4262) induit une régression massive de la masse tumorale préétablie. Cependant, l'efficacité de ce type de traitement n'est pas totale , puisque dans l'expérience décrite, seulement onze animaux sur les quatorze traités voient une régression tumorale complète macroscopique et microscopique . De plus, on injecte un petit nombre de cellules tumorales (4 x 10⁴ cellules) et le traitement (injection de cellules fibroblastiques produisant des particules virales TK⁺) est effectué très tôt ( dès le cinquième jour ) après l'inoculation des cellules tumorales . L'inconvénient principal reste néanmoins que l'on n'a pas observé dans ce modèle de mémoire immunitaire vis-à-vis d'une inoculation secondaire des mêmes cellules tumorales.

Le second modèle développe une approche relativement similaire à celle précitée . On traite des tumeurs hépatiques établies et macroscopiques par injection intratumorale de fibroblastes xénogéniques produisant des particules virales exprimant le gène TK du virus de l'Herpès et infectant sélectivement les cellules tumorales . Après une période de transduction du gène TK dans les cellules tumorales in vivo , la grande majorité de la masse tumorale est éliminée par un traitement par le Ganciclovir (GVC). Cependant les inconvénients principaux restent les mêmes , à savoir l'absence de garantie quant à l'élimination totale des cellules tumorales et la mémorisation.

Ces deux approches font appel à des cellules xénogéniques capables d'exprimer des particules virales pouvant transduire le gène TK dans les cellules tumorales . La seconde approche , comparée à celle développée par Culver et al., est plus efficace , car elle mime une situation de métastases hépatiques d'un cancer primaire du colon, et elle intervient sur un foyer tumoral bien développé et visible macroscopiquement .

Dans une autre technique décrite dans la demande PCT/US 92/O6 188 (UNIVERSITE DE ROCHESTER), on réinjecte à des patients atteints de cancers leurs propres cellules cancéreuses dans lesquelles un gène suicide a été introduit.

Les essais décrits dans cette demande montrent que le traitement par ce type de composition cellulaire transgénique, puis par la substance relative au gène suicide, permet d'entraîner la destruction dans l'organisme non seulement des cellules objets de la demande mais aussi des autres cellules cancéreuses. La destruction des cellules transgéniques dans l'organisme entraîne ainsi la destruction des autres cellules cancéreuses non transgéniques. Les compositions décrites dans cette demande ne comprennent pas de virus.

Enfin, une technique basée sur l'emploi combiné d'impulsions électriques et d'injections locales de cellules allogéniques ou xénogéniques secrétant l'Interleukine-2 a été récemment développée ( MIR et al., (1992) Compte-Rendu de l'Académie des Sciences de Paris , série III, 314, 539-544).

L'électrochimiothérapie consiste selon MIR et al. (Eur. J. Cancer 1991 , 27, 68-72 ) à injecter de manière locale de la bléomycine et à appliquer à proximité de la tumeur des impulsions électriques .

L'utilisation combinée de ces deux méthodes potentialise l'effet anti-tumoral observé pour chacune des deux méthodes utilisées individuellement .

Cependant , l'électrochimiothérapie présente l'inconvénient majeur , même en combinaison avec l'utilisation de cellules secrétant de l'Interleukine-2 , de n'être facilement applicable qu'à des tumeurs dont un foyer au moins est accessible .

Il ressort donc clairement de l'état de la technique analysé ci-dessus que les méthodes décrites ne sont pas toujours efficaces , en particulier à l'encontre de tumeurs établies , et qu'elles ne fournissent pas systématiquement de mémoire immunitaire spécifique de cette tumeur , ou ne permettent qu'un traitement local des tumeurs .

Les demandeurs se sont donc attachés à trouver une composition permettant d'obtenir d'une part une disparition rapide d'une tumeur , quelle que soit sa localisation , et d'autre part une immunisation spécifique et à long terme de l'organisme à l'encontre de la tumeur traitée .

Ils ont ainsi mis en évidence de manière surprenante que la combinaison de moyens de sécrétion d'immunomodulateurs, et de vecteurs conduisant de manière spécifique au suicide des cellules tumorales , permet le plus souvent d'obtenir une disparition rapide et définitive de la tumeur et une immunisation spécifique à long terme de l'organisme à l'encontre de cette tumeur .

La présente invention a donc pour objet une composition destinée à traiter les tumeurs d'organismes humains ou animaux et à les immuniser à l'encontre de cette tumeur, ladite composition comprenant en association synergique :
- des cellules , des virus , ou des bactéries exprimant de manière transitoire dans l'organisme au moins un gène leur permettant de produire in vivo un ou plusieurs immunomodulateurs , et
- des virus , ou des cellules produisant des virus , lesdits virus infectant, si possible , préférentiellement les cellules en division de l'organisme à traiter , et portant dans leur génome au moins un gène dont l'expression dans les cellules en division va entraîner leur mort .

Avantageusement , la composition telle que décrite ci-dessus est composée de cellules produisant in vivo un ou plusieurs immunomodulateurs et produisant en outre des virus portant dans leur génome au moins un gène dont l'expression dans les cellules en division va entraîner leur mort .

Les cellules utilisées dans ces compositions seront préférentiellement des cellules allogéniques ou xénogéniques, afin de permettre leur élimination des organismes traités . Elles pourront elles-mêmes être infectées par ou productrices de virus portant dans leur génome au moins un gène dont l'expression dans des cellules en division va entraîner leur mort, telle que définie ci-dessus .

Ladite composition peut ainsi comprendre des cellules dans lesquelles le virus utilisé pour infecter les cellules de l'organisme en division est responsable de la production de l'immunomodulateur .

Les immunomodulateurs sont avantageusement l'Interleukine-2 , l'Interleukine-4, l'Interleukine-7, le TNF ( Tumor Necrosis Factor ) l'Interféron-gamma, le GM-CSF ( Colony Stimulating Factor granulomonocytaire ) , seuls ou en combinaison .

Ces immunomodulateurs peuvent être exprimés, outre les cellules et bactéries citées ci-dessus , à partir de gènes portés sur le génome de virus, tels que des rétrovirus , des virus Pox ( virus de la vaccine , Canary Pox ), des Adénovirus ainsi que des virus défectifs associés aux Adénovirus (AAV).

Les bactéries peuvent être des bactéries intracellulaires produisant un immunomodulateur .

On notera que les immunomodulateurs peuvent être apportés par tout moyen, en plus des cellules, des bactéries ou des virus, permettant le relarguage d'immunomodulateurs dans l'organisme : un tel moyen est par exemple une micropompe greffée et libérant des immunomodulateurs dans l'organisme .

Les virus infectant préférentiellement les cellules en division de l'organisme traité et portant dans leur génome au moins un gène dont l'expression dans les cellules en division va entraîner leur mort , ou agents de lyse , sont préférentiellement des rétrovirus , des Pox virus ou des Adénovirus. Ces virus seront d'autant plus avantageux qu'ils peuvent présenter la propriété d'infecter ou tuer préférentiellement et en majeure partie les cellules de l'organisme qui se divisent .

Ils peuvent être apportés par des cellules choisies afin qu'elles soient éliminées rapidement dans l'organisme notamment allo- ou xénogéniques .

Le gène dont l'expression dans les cellules en division va entraîner leur mort est préférentiellement le gène d'une thymidine kinase ou d'une cytosine déaminase . La mort des cellules portant ces gènes sera induite en leur fournissant respectivement du Ganciclovir et de la 5-fluoro cytidine.

La présente invention est en outre relative à l'utilisation de la composition décrite ci-dessus pour la fabrication d'un médicament pour le traitement des tumeurs et cancers et pour l'immunisation de l'organisme à l'encontre de ces maladies.

De manière avantageuse , tous les éléments de la composition sont administrés simultanément . Ils peuvent être présentés simultanément ou indépendamment.

Il est aussi possible d'administrer de manière décalée dans le temps les deux composants principaux . Ainsi , avantageusement, on administre tout d'abord les virus, ou cellules produisant les virus , infectant préférentiellement les cellules de l'organisme en division et portant dans leur génome au moins un gène, dont l'expression dans les cellules en division va entraîner leur mort , puis les substrats, dont la métabolisation par les cellules en division va entraîner leur mort .

Cette phase d'élimination de la masse tumorale est suivie d'une phase d'immunisation de l'organisme à l'encontre de cette tumeur par administration des cellules , des virus ou des bactéries exprimant les immunomodulateurs.

La composition selon l'invention peut être introduite par tout moyen à la portée de l'homme du métier et en particulier par injection à la seringue sous imagerie médicale.

Ce traitement est avantageusement local mais peut tout aussi bien être systémique .

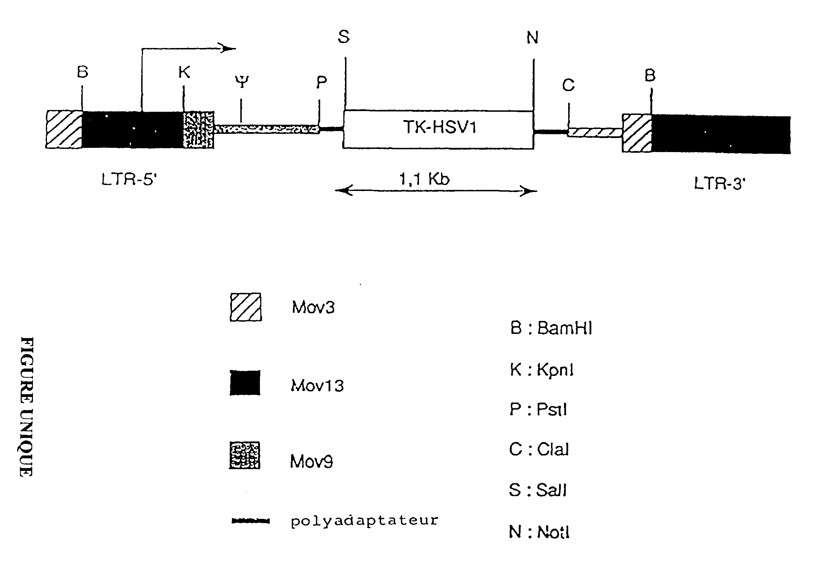

La présente invention est illustrée par l'exemple ci-après en référence à la figure unique annexée qui représente la carte du vecteur rétroviral pMTK .

### EXEMPLE :

### Traitement de souris présentant des tumeurs par des cellules sécrétant de l'Interleukine-2 et produisant le rétrovirus PMTK.

Des souris C56 BL/6 présentant des tumeurs ont été traitées par une composition contenant des cellules allogéniques produisant de l'Interleukine-2 et le rétrovirus PMTK qui porte le gène de la thymidine kinase du virus HSV1.

Les injections ont été effectuées près des tumeurs .

Le vecteur pMTK ( voir figure ) présente les caractéristiques suivantes :
LTR-5'
- Mov3 jusqu'au site BamH1 (-350 du départ de transcription ),
- Mov13 du site BamH1 (-350) jusqu'au site Kpn1 (+ 30)
- Mov9 du site Kpn1 (+30) jusqu'au site Pst1 (+560) (contient la séquence de conditionnement )
- La séquence non codante en 3' du site Cla1 (+ 7674) jusqu'à U3 (+ 7817) provient de Mov3.
   LTR-3'
- Mov3 jusqu'au site BamH1 positionné en 7910 (ou -350),
- Mov13 jusqu'à la fin de U5.

Une lignée cellulaire de conditionnement CRIP est co-transfectée par le plasmide pMTK (20µg) et le plasmide pWLNeO (1 µg). Plusieurs clones ont été isolés en sélection G418 .

Le titre du clone sélectionné est de 5.10⁵ particules infectieuses/ml, tel que mesuré par la capacité de conférer une résistance en HAT (Hypoxanthine, Aminoptérine, Thymidine) pour des cellules L TK⁻. L'infection des cellules L TK⁻ se réalise avec des dilutions successives du surnageant contenant les particules virales .

## Revendications

1. Composition destinée à traiter les tumeurs d'organismes humains ou animaux et à immuniser ces organismes à l'encontre des tumeurs, ladite composition comprenant :
- des cellules allogéniques ou xénogéniques pour les organismes traités, des bactéries, ou des virus, lesdits virus, cellules ou bactéries produisant in vivo un ou plusieurs immunomodulateurs, et
- des virus, ou des cellules allogéniques ou xénogéniques pour les organismes traités, produisant des virus, lesdits virus infectant préférentiellement les cellules en division de l'organisme traité et portant dans leur génome au moins un gène dont l'expression dans les cellules en division va entraîner leur mort.

2. Composition selon la revendication 1, caractérisée en ce que les cellules produisent in vivo un ou plusieurs immunomodulateurs et produisent en outre des virus portant dans leur génome au moins un gène dont l'expression dans les cellules en division va entraîner leur mort.

3. Composition selon la revendication 1, comprenant :
- des virus produisant in vivo un ou plusieurs immunomodulateurs, et
- des virus infectant préférentiellement les cellules en division de l'organisme traité et portant dans leur génome au moins un gène dont l'expression dans les cellules en division va entraîner leur mort.

4. Composition selon l'une des revendications 1 à 3, caractérisée en ce que les immunomodulateurs sont choisis parmi l'Interleukine-2, l'Interleukine-4, l'Interleukine-7, le TNF, l'Interféron-gamma et le GM-CSF, seuls ou en combinaison.

5. Composition selon l'une des revendications 1 à 4, caractérisée en ce que le gène dont l'expression entraîne la mort cellulaire exprime une thymidine kinase ou la cytosine déaminase.

6. Composition selon l'une des revendications 1 à 5, caractérisée en ce que l'immunomodulateur est le GM-CSF et le gène dont l'expression dans les cellules en division va entraîner leur mort, code pour une Thymidine Kinase.

7. Composition selon l'une des revendications 1 à 6, caractérisée en ce que les virus sont des rétrovirus, des Adénovirus, des virus associés aux Adénovirus ou des virus Pox.

8. Composition selon la revendication 1, comprenant :
- des rétrovirus produisant in vivo un ou plusieurs immunomodulateurs, et
- des rétrovirus exprimant une thymidine kinase.

9. Composition comprenant :
- des moyens permettant la libération in vivo d'un ou plusieurs immunomodulateurs dans un organisme humain, et
- des virus ou des cellules produisant des virus, lesdits virus portant dans leur génome au moins un gène codant pour une thymidine kinase ;
pour une utilisation simultanée, séparée ou espacée dans le temps.

10. Utilisation de la composition selon l'une des revendications 1 à 9 pour la fabrication d'un médicament pour le traitement des tumeurs et cancers et/ou l'immunisation de l'organisme à l'encontre de ces maladies.

11. Utilisation selon la revendication 10, caractérisée en ce que les différents composants de la composition sont administrés simultanément ou indépendamment.

12. Utilisation selon la revendication 10, caractérisée en ce que les virus, ou les cellules produisant les virus , infectant préférentiellement les cellules d'organismes en division, et portant dans leur génome au moins un gène dont l'expression dans les cellules en division va entraîner leur mort, sont administrés antérieurement ou simultanément à l'administration des cellules, virus ou bactéries, produisant les immunomodulateurs.
